# EUROPEAN PATENT APPLICATION

(11) **EP 1 169 964 A2**
(43) Date of publication of application: **09.01.2002**
(21) Application number: 01115400.2
(22) Date of filing: 26.06.2001
(51) Int. Cl.: A61B 5/00

(54) **Diagnosis system, diagnosis data producing method, information processing device, terminal device and recording medium used in the diagnosis data producing method**

(30) Priority: 27.06.2000 JP 2000192793
(71) Applicant: DRDC limited, Tokyo 108-0072 (JP); SCALAR CORP., Tokyo 151-0053 (JP)
(72) Inventor: Utsugi, Ryuichi, Tokyo 108-0072 (JP); Yamamoto, Masao, Tokyo 151-0053 (JP)
(74) Representative: Manitz, Finsterwald & Partner GbR

(57) **Abstract**

A diagnosis system includes a terminal T and a server S which are connected to each other through a network B. A user images a given position of user's body by an imaging device C connected to the terminal T and transmits image data obtained. by that imaging to the server S through the network. The server S compares the received image data with reference data of the server S and automatically diagnoses the user's health condition, to thereby produce diagnosis data. The diagnosis data is equivalent to a diagnosis result whom a doctor says a patient, to thereby realize an automatic diagnosis.

## Description

### BACKGROUND OF THE INVENTION

### 1. Field of the Invention

The present invention relates to a technique by which a diagnosis such as a depression diagnosis, an insomnia diagnosis or a drooping eyelid diagnosis is automatically conducted by using a computer.

### 2. Description of the Related Art

It is general that the diagnosis or medical treatment of a disease is conducted by a doctor. Also, it is general that a patient or a person to be diagnosed directly visits a medical doctor and faces the doctor to conduct the diagnosis and the medical treatment.

On the other hand, with an development in an infrastructure in recent years, a terminal is connected to a network such as an internet by even a general home or personal unit, thereby being capable of getting various information.

With the progress of the above technique, a technique in which a doctor and a patient whose terminals are connected to each other through the internet exchange voices and images at a real time, to thereby conduct a diagnosis and a medical treatment has been put in a practical use.

Incidentally, there are a diagnosis which is not so difficult and a medical treatment such as the skin aspect diagnosis that does not seriously affect the body or the life among the diagnoses and the medical treatments.

In the recent years where the life level has been improved, a demand for such slight diagnosis or medical treatment is extremely increased, and there exist many users who think to obtain the supply of the diagnosis or the medical treatment method related to the slight diagnosis or medical treatment without facing the medical doctor.

However, in the above-described technique that attaches importance to the real time property, although there is advantageous in that an accuracy in the diagnosis and the medical treatment is high, there is inconvenient in that the medical doctor and the patient must employ their terminals on time. Also, since the judgment of the diagnosis and the medical treatment is finally conducted by the doctor, there arises such a serious problem that the doctor must be ensured, and the above technique is unsuitable for the minor diagnosis or medical treatment.

### SUMMARY OF THE INVENTION

The present invention has been made to solve the above problem, and therefore an object of the present invention is to provide a diagnosis and medical treatment technique to which a network is applied where everybody can be subjected to a desired diagnosis and medical treatment at a desired time without requiring a medical doctor in principle.

In order to achieve the above object, according to the present invention, there is provided a diagnosis system, comprising: a terminal device that receives image data obtained by imaging from a given imaging means that repeatedly images a position to be imaged which is located at a given position of a user's body; and an information processing device which is connectable to said terminal device through a network; wherein said terminal device includes a transmitting means for transmitting the image data produced by imaging the position to be imaged by said imaging means to said information processing device through said network; wherein said image processing device includes a diagnosing means that conducts a given diagnosis on the basis of image data received from said terminal and produces diagnosis data pertaining to a diagnosis result; and wherein said information processing device produces the diagnosis data in a time series manner on the basis of a plurality of image data produced by imaging the position to be imaged at given time intervals.

In this diagnosis system, the information processing device automatically conducts the diagnosis based on the image information transmitted from a user's terminal by the user. Therefore, according to this diagnosis system, the diagnosis is conducted without troubling the doctor. As a result, the user is not required to set a time with the doctor and is easily diagnosed whenever and wherever.

Also, in this diagnosis system, a plurality of image data is produced at the same imaging position by repeatedly imaging a given position to be imaged in a user's body, and the image processing device that has received the image data produces the diagnosis data with respect to the respective image data. This corresponds to the actual diagnosis in which a given position of the user is fixed-point observed to follow its development. That is, according to the present invention, since the given position to be imaged is observed at given time intervals, the diagnosis high in the precision is conducted while being simple. The diagnosis data is produced in accordance with the time series on the basis of the received image data. The diagnosis data may be produced on the basis of all of the received image data or may be produced on the basis of only the required image data.

For example, if the above-described diagnosis data is returned to the user, the use can apply the diagnosis data to the user's health management. For example, a medicine or cosmetics which is judged to be required on the basis of the diagnosis data can be purchased by the user.

Also, the diagnosis data can be utilized by the manufacturers or the distributors of the medicine, the cosmetics, medical apparatuses or beauty implements. For example, if the diagnosis data is sent to the distributors of the medicine or the cosmetics, the diagnosis data can be utilized by the distributors. For example, a system can be constructed in which the medicine or the cosmetics are delivered from the distributor that has received the diagnosis data automatically or in response to a purchase request from the user. In this case, the user needs to make some contract with a person who manages the information processing device, and with such a contract, if the user conducts a simple process for photographing and transmitting given image data at home, the user can get the medicine, the cosmetics, medical apparatuses or beauty implements which meet the user's health condition while being at home.

In the present specification, the image includes not only a static image but also a moving image.

In the system, the image data is transmitted from the information processing device from the user's terminal, but the oral consultation data related to the diagnosis contents and including given character information may be also transmitted to the information processing device. The oral consultation data may include, for example, information on the age and sex of the user and information on the life environments of the user. The information that cannot be included in the image data is included in the oral consultation data, thereby being capable of enhancing a precision in the diagnosis.

In the present specification, the character information includes the information on marks used for expressing the information related to the diagnosis contents in addition to general character information such as text data. For example, the information produced by selecting one from a plurality of options is called "character information" in the present specification.

In this case, the information processing device produces the diagnosis data taking not only the image data but also the oral consultation data into consideration.

In the above information processing device, not only the diagnosis data is produced, but also prescription data related to the prescription that meets the diagnosis result may be produced on the basis of the diagnosis data. In this case, the prescription data according to the time series is produced on the basis of the diagnosis data produced according to the time series. With this operation, a series of works including the diagnosis and the prescription which are made by the doctor can be automated. The prescription data is available to the user, or the manufacturers and the distributors of the medicine, the cosmetics, medical apparatuses or beauty implements as in the above-described diagnosis data.

A terminal device that constitutes a part of the above system in accordance with the present invention is as follows.

That is, there is provided a terminal device which is connectable through a network to an image processing device having a diagnosing means that conducts a given diagnosis on the basis of image data received through the network to produce diagnosis data pertaining to a diagnosis result, said terminal device comprising: a transmitting means that receives, from a given imaging means that repeatedly images a position to be imaged which is located at a given position of a user' s body, the image data produced by imaging the position to be imaged with the use of said imaging means by the user to transmit the image data to the information processing device through said network.

Also, the terminal device is designed as follows in the case where an intention is made to use the above-described oral consultation data.

That is, there is provided a terminal device which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result, said terminal device comprising: a transmitting means that receives, from a given imaging means that repeatedly images a position to be imaged which is located at a given position of a user' s body, the image data produced by imaging the position to be imaged with the use of said imaging means by the user and the diagnosis data and transmits the image data and the diagnosis data to the information processing device through said network.

Also, a terminal device that constitutes the network according to the present invention can execute the following method.

That is, there is provided a method of transmitting diagnosis information which is executed by a terminal device that is connectable through a network to an information processing device having a diagnosing means that conducts a given diagnosis on the basis of image data received through the network to produce diagnosis data pertaining to a diagnosis result, said method comprising the steps of: receiving image data produced, from a given imaging means that repeatedly images a position to be imaged which is located at a given position of a user's body, the image data produced by imaging the position to be imaged with the use of said imaging means by the user; and transmitting the image data to the information processing device through the network.

Also, the terminal device that constitutes the network according to the present invention can execute the following method in the case where an intention is made to also use the above-described oral consultation data.

That is, there is provided a method of transmitting diagnosis information which is executed by a terminal device which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result, said method comprising the steps of: receiving image data produced, from a given imaging means that repeatedly images a position to be imaged which is located at a given position of a user's body, the image data produced by imaging the position to be imaged with the use of said imaging means by the user and the diagnosis data; and transmitting the image data and the diagnosis data to the information processing device through the network.

Also, the terminal device and the method of transmitting the diagnosis information can be readily realized by using a home computer, a computer only for a game having a communicating means, or the like by allowing the following program recorded in a given recording medium to be read in the computer.

The above recording medium is exemplified below.

That is, there is provided a recording medium that records, in a computer readable manner, a program for allowing a terminal device with a computer which is connectable through a network to an information processing device having a diagnosing means that conducts a given diagnosis on the basis of image data received through the network to produce diagnosis data pertaining to a diagnosis result to function as a transmitting device, said computer executing said program comprising the steps of: receiving data produced, from a given imaging means that repeatedly images a position to be imaged which is located at a given position of a user's body, the image data produced by imaging the position to be imaged with the use of said imaging means by the user; and transmitting the image data to the information processing device through the network.

Also, in the case where the network according to the present invention is intended to also use the above-described oral consultation data, the recording medium can be structured as follows.

That is, there is provided a recording medium that records, in a computer readable manner, a program for allowing a terminal device with a computer which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result to function as a transmitting device, said computer executing said program comprising the steps of: receiving image data produced, from a given imaging means that repeatedly images a position to be imaged which is located at a given position of a user's body, the image data produced by imaging the position to be imaged with the use of said imaging means by the user and the diagnosis data; and transmitting the image data and the diagnosis data to the information processing device through the network.

On the other hand, the information processing device that constitutes a part of the above system according to the present invention is stated below.

That is, there is provided an information processing device that is connectable through a network to a terminal device having a transmitting means that receives, from a given imaging means that repeatedly images a position to be imaged which is located at a given position of a user' s body, the image data produced by imaging the position to be imaged with the use of said imaging means by the user to transmit the image data through said network, said information processing device comprising: wherein said image processing device includes a diagnosing means that conducts a given diagnosis on the basis of image data received from said terminal and produces diagnosis data pertaining to a diagnosis result; and wherein said information processing device produces the diagnosis data in a time series manner on the basis of a plurality of image data produced by imaging the position to be imaged at given time intervals.

In the case where an intention is made to also use the above-described oral consultation data, the information processing device is structured, for example, as follows.

That is, there is provided An information processing device which is connectable through a network to a terminal device having a transmitting means that receives, from a given imaging means that repeatedly images a position to be imaged which is located at a given position of a user' s body, image data produced by imaging the position to be imaged by the user and oral consultation data pertaining to the diagnosis contents and including given character information and transmits the image data and the diagnosis data through said network, said information processing device comprising: a diagnosing means that conducts a given diagnosis on the basis of the image data and the oral consultation data received from said terminal and produces diagnosis data pertaining to a diagnosis result; wherein said information processing device produces the diagnosis data in a time series manner on the basis of a plurality of image data and oral consultation data produced by imaging the position to be imaged at given time intervals.

Also, in the case where the information processing device also produces the above-described prescription data, the information processing device is structured as follows. That is, there is provided the information processing device further comprising a prescribing means for producing prescription data pertaining to a prescription suitable for the diagnosis result; wherein said prescribing means produces the prescription data in accordance with the time series on the basis of the diagnosis data produced in accordance with the time series.

Both of the above information processing devices can be structured, for example, as follows: That is, there is provided the information processing device wherein said diagnosing means comprises: an image processing means for conducting a given image process on the image data to produce comparison image data if occasion demands; reference data recording means for recording reference data for diagnosis; and a judging means for comparing the comparison image data with the reference data to conduct the given diagnosis.

In the case where an intention is made to also use the above-described oral consultation data, the diagnosing means of the information processing device comprises an image processing means for conducting a given image process on the image data to produce comparison image data; reference data recording means for recording reference data for diagnosis; and a judging means for comparing the comparison image data and the oral consultation data with the reference data to conduct the given diagnosis.

The reference data includes data necessary for conducting plural kinds of diagnoses. In this case, if correspondence information indicative of which diagnosis the image corresponds to is added to the image data, said judging means can be designed to conduct the plural kinds of diagnoses indicated by the correspondence information on the basis of the correspondence information received together with the image data. With this arrangement, plural kinds of diagnoses can be accurately executed by one system.

Also, the reference data may include data for conducting at least one of a depression diagnosis, an insomnia diagnosis, a drooping eyelid diagnosis, an autonomic imbalance diagnosis, a skin aspect diagnosis and a hair diagnosis. Then, said judging means conducts at least one of the depression diagnosis, the insomnia diagnosis, the drooping eyelid diagnosis, the autonomic imbalance diagnosis, the skin aspect diagnosis and the hair diagnosis indicated by the correspondence information.

In this structure,

Also, correspondence information indicative of which diagnosis the image corresponds to is added to the image data, and said judging means may conduct any one of the depression diagnosis, the insomnia diagnosis, the drooping eyelid diagnosis, the autonomic imbalance diagnosis, the skin aspect diagnosis and the hair diagnosis indicated by the correspondence information on the basis of the correspondence information received together with the image data.

The information processing device that constitutes the network according to the present invention can be executed by the following method.

That is, there is provided a method of producing diagnosis data which is executed by an information processing device which is connectable through a network to a terminal device having a transmitting means that receives, from a given imaging means that repeatedly images a position to be imaged which is located at a given position of a user' s body, the image data produced by imaging the position to be imaged by the user to transmit the image data through said network, said method comprising: receiving the image data from said terminal; and conducting a given diagnosis on the basis of the image data to produce the diagnosis data pertaining to the diagnosis result on the basis of the diagnosis result; wherein said receiving step and said conducting step are repeatedly conducted on a plurality of image data produced by imaging the position to be imaged at given time intervals by the user to produce the diagnosis data in accordance with the time series.

In the case where an intention is made to also use the above-described oral consultation data, the information processing device that constitutes the network according to the present invention can be executed by the following method.

That is, there is provided a method of producing diagnosis data which is executed by an information processing device which is connectable through a network to a terminal device having a transmitting means that receives, from a given imaging means that repeatedly images a position to be imaged which is located at a given position of a user' s body, the image data produced by imaging the position to be imaged by the user and oral consultation data pertaining to the diagnosis contents and including given character information to transmit the image data and the oral consultation data through said network, said method comprising: receiving the image data and the oral consultation data from said terminal; and conducting a given diagnosis on the basis of the image data and the oral consultation data to produce the diagnosis data pertaining to the diagnosis result on the basis of the diagnosis result; wherein said receiving step and said conducting step are repeatedly conducted on a plurality of image data produced by imaging the position to be imaged at given time intervals and the oral consultation data by the user to produce the diagnosis data in accordance with the time series.

The information processing device and the method of producing the diagnosis data can be readily realized by using a general-purpose computer such as a home computer by allowing the following program recorded in a given recording medium to be read in the computer.

That is, there is provided a recording medium that records, in a computer readable manner, a program for allowing an information processing device with a computer which is connectable through a network to a terminal device having a transmitting means that receives, from a given imaging means that repeatedly images a position to be imaged which is located at a given position of a user' s body, the image data produced by imaging the position to be imaged with the use of said imaging means by the user to transmit the image data through said network to function as a diagnosis data producing device, said computer executing said program comprising the steps of: receiving the image data from said terminal; and conducting a given diagnosis on the basis of the image data and the oral consultation data to produce the diagnosis data pertaining to the diagnosis result; wherein said receiving step and said conducting step are repeatedly conducted on a plurality of image data produced by imaging the position to be imaged at given time intervals to produce the plurality of diagnosis data in accordance with the time series.

Also, in the internet according to the present invention, in the case where an intention is made to also use the above-described oral consultation data, the recording medium is structured, for example, as follows:

That is, there is provided a recording medium that records, in a computer readable manner, a program for allowing an information processing device with a computer which is connectable through a network to a terminal device having a transmitting means that receives, from a given imaging means that repeatedly images a position to be imaged which is located at a given position of a user' s body, the image data produced by imaging the position to be imaged with the use of said imaging means by the user and oral consultation data pertaining to the diagnosis contents and including given character information to transmit the image data through said network to function as a diagnosis data producing device, said computer executing said program comprising the steps of: receiving the image data and the oral consultation data from said terminal; and conducting a given diagnosis on the basis of the image data and the oral consultation data to produce the diagnosis data pertaining to the diagnosis result; wherein said receiving step and said conducting step are repeatedly conducted on a plurality of image data produced by imaging the position to be imaged at given time intervals and the oral consultation data to produce the plurality of diagnosis data in accordance with the time series.

### BRIEF DESCRIPTION OF THE DRAWINGS

These and other objects and advantages of this invention will become more fully apparent from the following detailed description taken with the accompanying drawings in which:
Fig. 1 is a diagram schematically showing the entire structure of a diagnosis system in accordance with one embodiment of the present invention;
Fig. 2 is a perspective view showing a use mode of a positioning implement 10 available in imaging by a terminal of the diagnosis system shown in Fig. 1;
Fig. 3 is a functional block diagram showing the structure of a terminal included in the diagnosis system shown in Fig. 1;
Fig. 4 is a functional block diagram showing the structure of a server included in the diagnosis system shown in Fig. 1;
Fig. 5 is a diagram showing an example of an image displayed on a display device of the terminal when oral consultation data is inputted to the terminal;
Figs. 6A to 6C are diagrams showing examples of an image indicated by comparison image data used in a drooping eyelid diagnosis;
Fig. 7 is a diagram for explaining an example of the contents of reference data used in the drooping eyelid diagnosis;
Fig. 8 is a diagram for explaining an example of the contents of prescription reference data used in the drooping eyelid diagnosis;
Fig. 9 is a diagram for explaining an example of the contents of reference data used in a depression diagnosis (1);
Fig. 10 is a diagram for explaining an example of the contents of the prescription reference data used in the depression diagnosis (1);
Figs. 11A to 11C are graphs for explaining examples of the contents of the reference data used in a depression diagnosis (2), respectively;
Fig. 12A and 12B are diagrams showing examples of an image indicated by comparison image data used in a hair diagnosis, respectively;
Fig. 13 is a diagram for explaining an example of the contents of the reference data used in the hair diagnosis;
Fig. 14 is a diagram for explaining an example of the contents of the reference data used in the hair diagnosis;
Fig. 15 is a diagram showing an example of an image indicated by comparison image data used in a skin aspect diagnosis; and
Fig. 16 is a diagram for explaining an example of the contents of the reference data used in the skin aspect diagnosis.

### DETAILED DESCRIPTION OF THE PREFERRED EMBDOIMENTS

Now, a description will be given in more detail of preferred embodiments of the present invention with reference to the accompanying drawings.

### (Entire Structure)

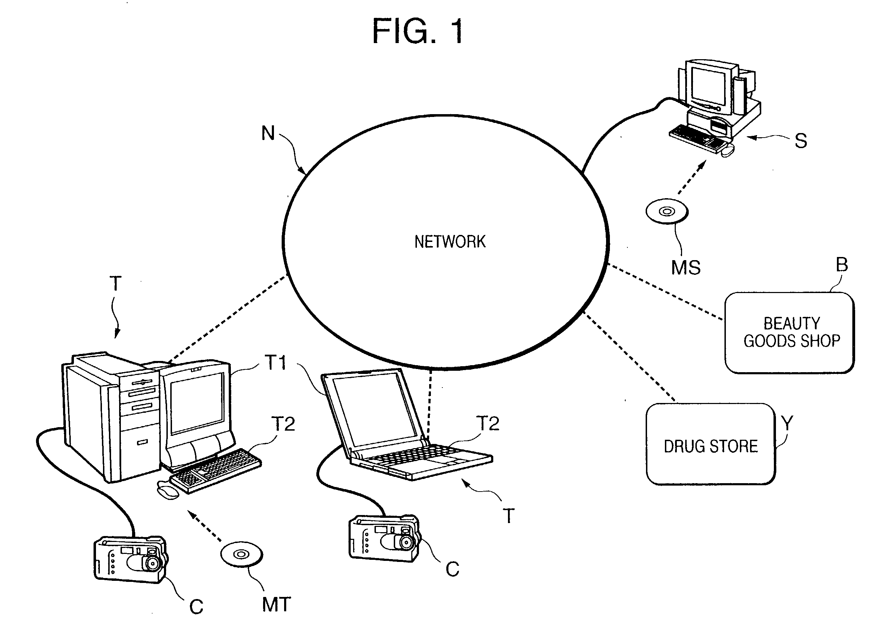

A diagnosis system embodied by this embodiment is shown in Fig. 1.

The diagnosis system includes a terminal T as a terminal device of the present invention and a server S as an information processing device of the present invention, and also includes a beauty goods shop B and a drugstore Y. They are connected to each other through a network N made up of an internet or an intranet.

### (Structure of terminal)

Then, the terminal T will be described. The terminal T according to this embodiment is made up of a home computer although the present invention is not limited to this.

The terminal T includes a given display device T1, an input device T2 made up of a keyboard, a mouse and so on for inputting oral consultation data which will be described later, and an imaging device C made up of a digital camera and so on.

The imaging device C corresponds to an imaging means of the present invention. The imaging device A is so designed as to produce image data by imaging. The terminal T can receive the above-described image data through an input terminal connected with a cable that connects the terminal T and the imaging device C.

The imaging device C is so used to image a given position to be imaged which is a part of a user's body by plural times as will be described later. That is, the imaging device C is required to image the same position to be imaged at given time intervals by plural times. In order to image the same position to be imaged by plurality times, imaging can be conducted referring to the relative position to a characteristic portion of the body such as a mole, eyes or a nose. For example, a portion 5 mm right from a specific mole is set as the position to be imaged and can continue to be imaged.

If occasion demands in the case of imaging, the imaging is repeated while not only the position but also the magnification of imaging is fixed.

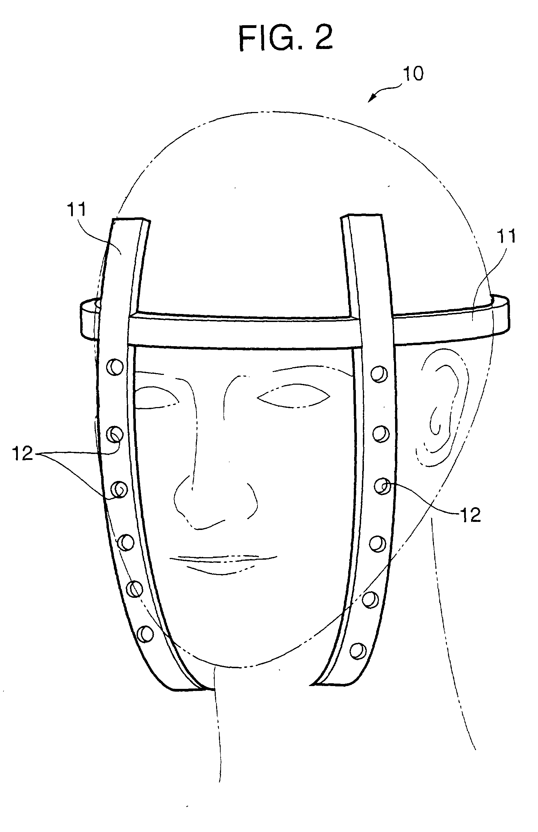

In order to image the same position to be imaged, an implement for subserving the imaging, for example, a headgear-shaped positioning implement 10 shown in Fig. 2 can be employed. The positioning implement 10 is designed in such a manner that the user wears the positioning implement 10 on his head in a given direction, and the imaging device C is fitted to a given one of a large number of positioning apertures 12 defined in frames 11 each of which extends longitudinally and laterally, thereby being capable of positioning the camera to a specific position. The positioning implement 10 may be fixed to a given portion of his face, for example, at three points. This makes it possible to more accurately position the imaging device C. The accurate positioning may be conducted by using the above subserving implement before the imaging is conducted.

The computer that serves as the terminal T includes a disk drive. A recording medium MT made up of a CD-ROM or a DVD-ROM according to the present invention is inserted into the disk drive so as to read a program stored in the recording medium according to the present invention. The computer has the respective functions required for the terminal T of the present invention by installing the program of the present invention through the above-described process.

The recording medium MT in this embodiment is distributed by a manager of the server S who manages this system, or the like although the present invention is not limited to this. It is not always necessary that the above-described program is installed in the computer through the network, but the program may be installed in the computer through a delivering process via the network.

The program can provide the computer with a function as the terminal T of the present invention by its single unit, or may provide the computer with a function of the terminal T of the present invention in cooperation with OS installed in the computer.

With the execution of the above-described program, the CPU that is built in the terminal T of the present invention constitutes the following functional blocks.

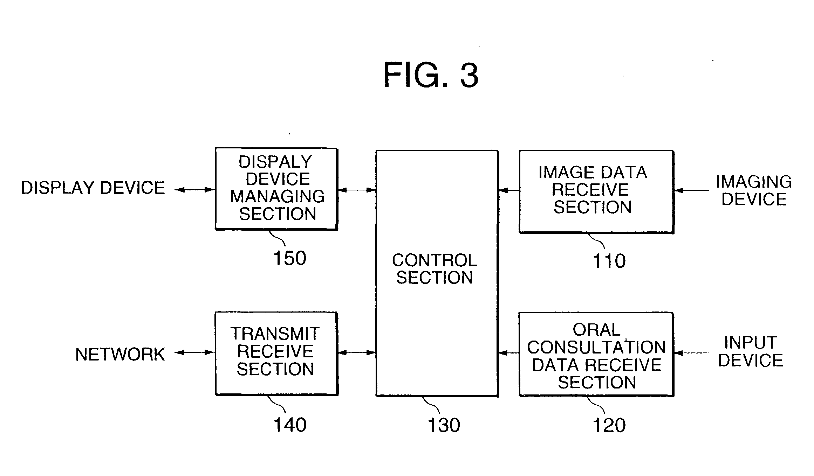

That is, as shown in Fig. 3, the terminal T is made up of an image data receive section 110, an oral consultation data receive portion 120, a control section 130 and a transmit/receive section 150.

The image data receive section 110 receives given image data produced by imaging through the imaging device C. In this embodiment, the image data receive section 110 is so designed as to receive the image data through the cable that connects the imaging device C and the terminal T. The image data receive section 110 may be so designed as to receive the image data produced in the imaging device C, and may be so structured as to receive the image data through a memory card or other external recording medium.

The oral consultation data producing section 120 produces the oral consultation data in response to an input that is conducted by the user through the input device. The oral consultation data is directed to information related to the contents of the diagnosis and also includes character information.

The oral consultation data can include information related to, for example, the age, the sex and the subjective symptom of the user and information related to date when the image data has been produced (imaging has been made), a life rhythm and a life pattern. That is, the oral consultation data can include any information necessary for the diagnosis if the information is related to character data. Also, the oral consultation data can be appropriately changed in accordance with the kind of the diagnosis contents. For example, in the case where the skin aspect diagnosis is made, the oral consultation data can include information related to the kind of cosmetics in use.

The oral consultation data is generally inputted as the character information from the keyboard that constitutes the input device T. For example, the oral consultation data can be produced by freely hitting characters by the user. A function of supplying the format of the diagnosis to be conducted to the user is provided in the control section 130 formed by execution of the above-described program, and the use can input the oral consultation data in that format. Also, the control section 130 is provided with a function of displaying, in the display device T1, a plurality of options related to information required at the time of conducting the subject diagnosis. The oral consultation data is inputted by selecting the options displayed on the display device T1 by the user.

The control section 130 manages the above-described image data receive section 110 and oral consultation data receive section 120 and a transmit/receive section 140 and a display device managing section 150 which will be described later.

For example, the control section 130 determines the image data and the oral consultation data to be transmitted from the transmit/receive section 140 on the basis of the input contents of the user, and an image displayed on the display device T1.

Also, the control section 130 has a function of producing the correspondence information which is data indicating that for which diagnosis the received image data is on the basis of an input from the above-described input device T2 or the like and adding the correspondence information to the image data.

The transmit/receive section 140 executes a communication between the terminal T and the network N. The transmit/receive section 140 is so designed as to transmit at least the image data (and added correspondence information) and the oral consultation data to the server S through the network N.

In this embodiment, the transmit/receive section 140 is so designed as to receive the diagnosis data produced by the server S (both of diagnosis data and prescription data, or prescription data depending on the occasion) which will be described later from the server S through the network N.

The display device managing section 150 is so designed as to produce display data for displaying a desired image on the display device T1. The display device managing section 150 is so designed as to produce the display data under the management by the control section 130.

### (Structure of Server)

Subsequently, the server S will be described. In this embodiment, the server S is made up of a general computer.

The server S includes a given display device and a given input device made up of a keyboard, a mouse and so on.

The computer that serves as the server S includes a disk drive.

A recording medium MT made up of a CD-ROM or a DVD-ROM according to the present invention is inserted into the disk drive so as to read a program stored in the recording medium according to the present invention. The computer has the respective functions required for the server S of the present invention by installing the program of the present invention through the above-described process.

The program can provide the computer with a function as the information processing device of the present invention by its single unit, or may provide the computer with a function of the information processing device of the present invention in cooperation with OS installed in the computer.

With the execution of the above-described program, the CPU built in the server S of the present invention constitutes the following functional block.

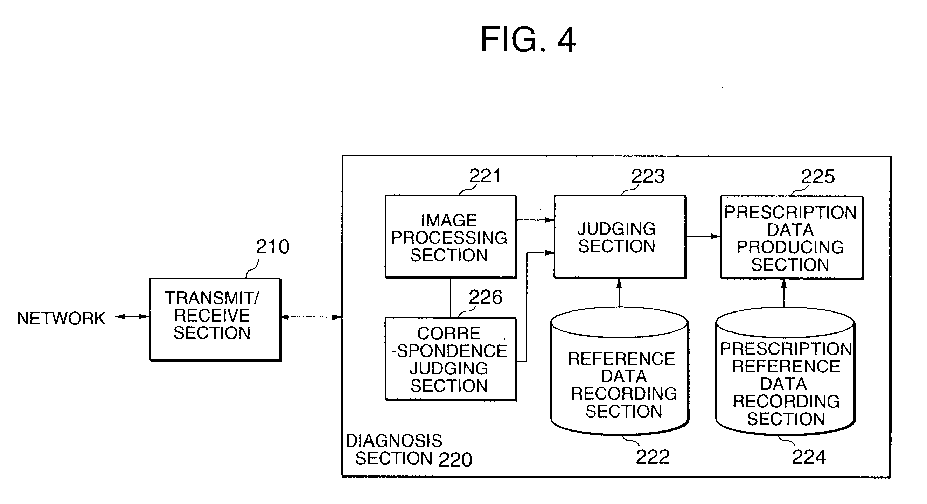

That is, as shown in Fig. 4, the server S includes a transmit/receive section 210 and a diagnosis section 220.

The transmit/receive section 210 is so designed as to execute a communication between the terminal T and the network N. The transmit/receive section 210 is so designed as to receive at least the image data and the oral consultation data from the terminal T.

In this embodiment, the transmit/receive section 210 can transmit the diagnosis data (both of prescription data and diagnosis data, or prescription data depending on the occasion) to a beauty salon B, the drugstore Y or the terminal Y used by the user through the network N. The transmission of the prescription data to the beauty salon B, the drugstore Y or the terminal T is selectively conducted with respect to one or plural portions.

The diagnosis section 220 has both functions of the diagnosing means and the prescribing means of the present invention. The diagnosis section 220 conducts a given diagnosis on the basis of the above-described image data and oral consultation data which have been received from the terminal T through the network N, and produces the prescription data related to a prescription that meets the diagnosis result.

In more detail, the diagnosis section 220 includes an image processing section 221, a reference data recording section 222, a judging section 223, a prescription reference data recording section 224, a data producing section 225 and a correspondence judging section 226.

The image processing section 221 corresponds to the image processing means of the present invention, and has a function of producing correspondence image data by conducting a given image process on the image data.

The reference data recording section 222 corresponds to a part of the reference data recording means according to the present invention, and records the reference data necessary for producing the prescription data. The reference data recording section 222 may record the reference data for conducting at least one diagnosis. In this embodiment, the reference data recording section 222 records the reference data for a drooping eyelid diagnosis, a depression diagnosis, an insomnia diagnosis, an autonomic imbalance diagnosis, a skin aspect diagnosis and a hair diagnosis, and said judging means conducts at least of the depression diagnosis, the insomnia diagnosis, the drooping eyelid diagnosis, the autonomic imbalance diagnosis, the skin aspect diagnosis and the hair diagnosis.

The judging section 223 functions as the judging means according to the present invention in cooperation with a prescription data producing section 225 which will be described later. The judging section 223 compares the comparison image data produced in the above-described image processing section 221 with the reference data that meets a desired diagnosis and produces the diagnosis data related to the diagnosis result taking the above-described oral consultation data into consideration.

Also, data required in producing the prescription data on the basis of the above-described diagnosis data is recorded in the prescription reference data recording section 224. In this embodiment, the prescription data recording section 224 records data related to the prescriptions pertaining to the drooping eyelid diagnosis, the depression diagnosis, the insomnia diagnosis, the autonomic imbalance diagnosis, the skin aspect diagnosis and the hair diagnosis as the prescription data therein although the present invention is not limited to this.

The prescription data producing section 225 corresponds to the prescribing means according to the present invention. The prescription data producing section 225 receives the above-described diagnosis data and reads appropriate data from the prescription reference data recording section 225, to thereby produce the prescription data. The prescription data is data related to a prescription that meets the present status indicated by the diagnosis data, and includes, for example, an advice for recovering the present health state, an advice for recovering the skin aspect or information related to those advices. The prescription data includes the contents corresponding to the respective diagnoses.

The correspondence judging section 226 analyzes the correspondence information transmitted from the terminal T which is added to the image data and determines a diagnosis to be conducted on the image data. In this embodiment, because the respective diagnoses of the drooping eyelid diagnosis, the depression diagnosis, the insomnia diagnosis, the autonomic imbalance diagnosis, the skin aspect diagnosis and the hair diagnosis can be conducted, the correspondence judging section 226 determines which of those diagnoses should be conducted on the basis of the correspondence information. The determination is transmitted to the image processing section 221 and the judging section 223. The image processing section 221 conducts an appropriate image process on the basis of the correspondence information, and the judging section 223 judges an appropriate diagnosis.

### (Flow of Producing Prescription Data)

A flow of producing the prescription data will be described in each of the drooping eyelid diagnosis, the depression diagnosis, the insomnia diagnosis, the autonomic imbalance diagnosis, the skin aspect diagnosis and the hair diagnosis. In the respective descriptions of the depression diagnosis and the subsequent diagnoses, duplicate portion of the description of the drooping eyelid diagnosis will be appropriately omitted.

### (Drooping Eyelid Diagnosis)

The drooping eyelid means that an eyelid hangs down due to the abnormality of an eyelid levator or the eyelid chorda which is a muscle that hangs the eyelid or the connection failure of the eyelid levator or the eyelid chorda and a tarsal. The drooping eyelid appears as various mental and physical disease symptoms. Therefore, because the various diseases may be prevented by early detecting the drooping eyelid, the detection of the drooping eyelid is very significant.

The diagnosis of the drooping eyelid is conducted, for example, as follows.

The user first inputs the declaration of his intention that he determines to be subjected to the drooping eyelid diagnosis through the input device T2. As a result, the control section 140 produces correspondence information indicating that a diagnosis conducted on the basis of image data which will be inputted later is the drooping eyelid diagnosis.

Then, the user images his eyes by the imaging device C and produces the image data related to the image of his eyes. The image of his eyes is set within a constant range including the user' eyes.

The terminal T receives the image data produced by imaging the his eyes by the imaging device C by the image data receive section 110. The data is transmitted to the transmit/receive section through the control section 140. Also, the control section 140 adds the above-described correspondence information to the image data.

The transmit/receive section 140 is controlled by the control section 130 in response to a user's demand which is inputted by the operation of the terminal T, and transmits the image data added with the correspondence information to the server S through the network N.

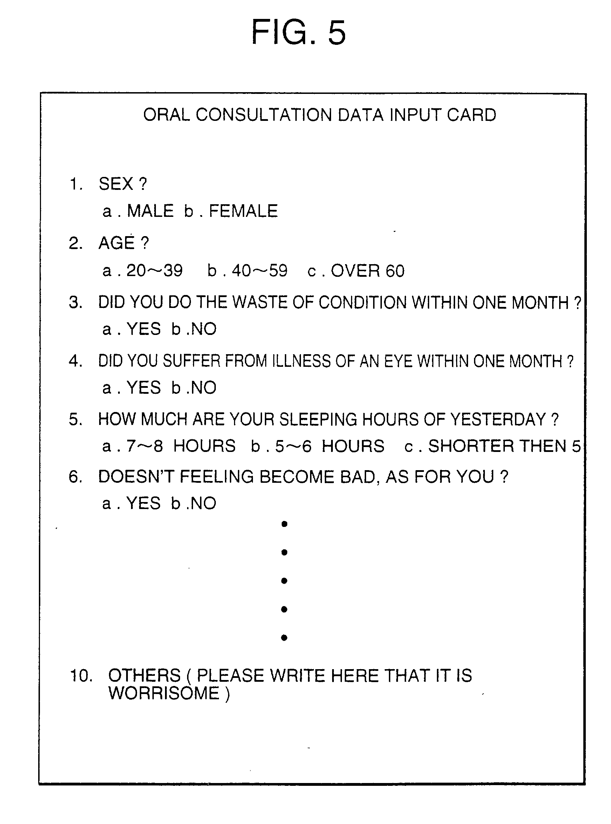

As occasion demands, the user conducts the operation for inputting the oral consultation data including the character information through the input device T2, to thereby produce the oral consultation data. In this embodiment, in inputting the oral consultation data, an image, for example, shown in Fig. 5 is displayed in the display device T1. The image is displayed on the display device T1 on the basis of display data that is produced by the display device managing section 150 under the control of the control section 130.

The user selects one option from options a and b (or a, b and c), for example, in examples (1) to (6), and inputs a document through a keyboard or the like in an example (10), to thereby input the oral consultation data. In the case where the oral consultation data has been inputted, the terminal T receives the above-described oral consultation data through the oral consultation data receive section 120. The data is transmitted to the transmit/receive section through the control section 140.

The transmit/receive section 140 is controlled by the control section 130 in response to the user's demand inputted through the operation of the terminal T, and adds the oral consultation data to the image data to transmit those data to the server S.

The server S receives the image data (or both of the image data and the oral consultation data) transmitted from the terminal T through the network N by the transmit/receive section 210, and conducts the diagnosis as to the drooping eyelid diagnosis on the basis of the data.

The image data related to the eyes received from the transmit/receive section 210 is transmitted to the diagnosing section 220.

First, the correspondence judging section 226 analyzes the correspondence information added with the image data and determines that a diagnosis to be conducted on the basis of the transmitted image data is the drooping eyelid diagnosis on the basis of the correspondence information. This determination is transmitted to the image processing section 221 and the judging section 223 to ensure an appropriate process in the image processing section 221 and an appropriate judgment in the judging section 223.

Also, the image data is transmitted to the image processing section 221. As occasion demands, the image processing section 221 conducts a given image process on the image data to produce comparison image data.

For example, a given threshold value of brightness is set to subject the image to a binary or ternary coding, thereby being liable to conduct the subsequent judgment as to the image of the eyes. Also, an edge emphasis or a contour line process due to moire may be conducted.

Also, the image processing section 221 may conduct a color correction process. Since the image data is frequently different from the natural image due to a difference in the specification between the imaging devices C or a difference in illumination at the time of imaging, the color correction is conducted, thereby being capable of conducting an accurate diagnosis. For example, a seal or the like on which a given reference color is stuck at a given arrangement is distributed to the user in advance, and the user is obliged to stick the seal to an imaging area to conduct imaging. Then, the reference color within the seal which is reflected in the image is adjusted to a color which is prepared in advance, and this process is applied to the entire image. The color correction process may be conducted at the terminal T of the user side. In this case, the unified image data which has been subjected to the color correction is transmitted to the server S. In this case, the terminal T is required to provide a means for conducting the color correction, for example, to provide a function of the above-described control section 130.

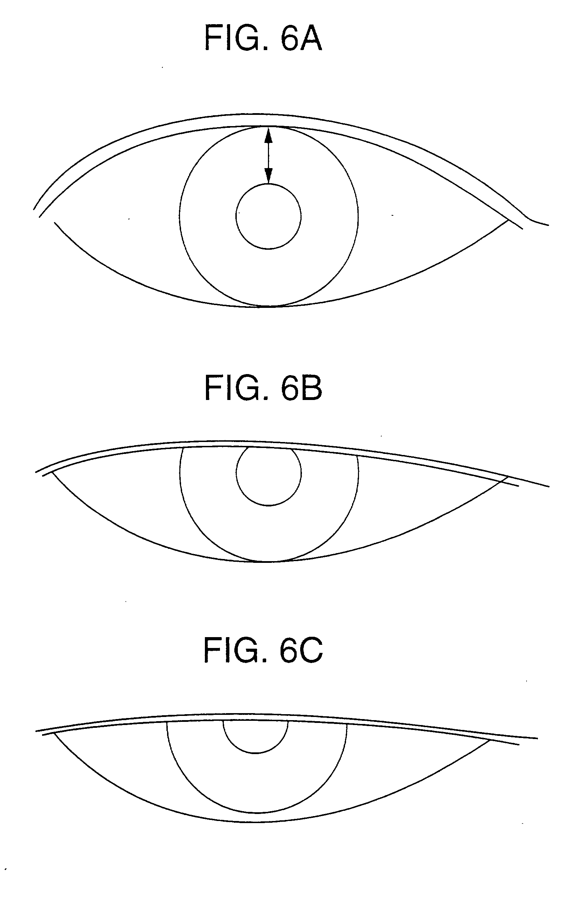

In this embodiment, the image of the user's eyes is converted into an image of a diagram shown in Figs. 6A to 6C which definitely shows only the contour of the eyes, the boundary of the white and black (gray) of the eye, and the contour of pupil, to produce the comparison image data related to the image.

The comparison image data which has been subjected to the above-described process as occasion demands is then transmitted to the judging section 223. The judging section 223 compares the comparison image data with the reference data read from the reference data recording section 222 to conduct a judgment on the basis of the comparison image data. This judgment corresponds to the diagnosis made by the doctor.

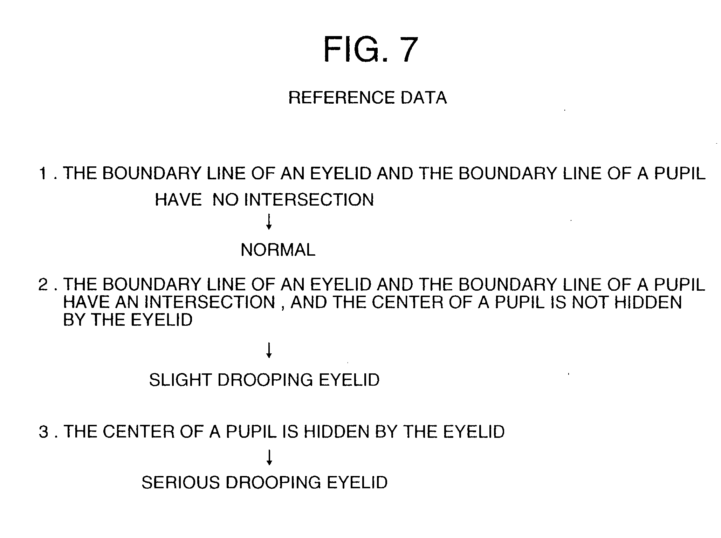

In the judgment, there is used the reference data related to the drooping eyelid diagnosis recorded in the reference data recording section 222. The reference data is not particularly restricted if it is used to conduct an appropriate judgment as to the drooping eyelid. The reference data is, for example, shown in Fig. 7.

The judging section 223 conducts the judgment by judging which reference of states 1 to 3 shown in Fig. 7 the image based on the comparison image data meets, and produces the diagnosis data. For example, if the eyelid is set within a range indicated by an arrow in fig. 6A, a judgment is made that the image meets the reference 1 corresponding to "normal", and in case of Fig. 6B, a judgment is made that the image meets the reference 2 corresponding to "slight drooping eyelid". In case of Fig. 6C, a judgment is made that the image meets the reference 3 corresponding to "serious drooping eyelid". The data related to those judgment results are diagnosis data.

If the following contents are added to the comparison image data, the diagnosis can be more accurately produced on the basis of the image.

That is, in case of a user whose eyebrows are elevated, since it is hard that the symptom of the drooping eyelid is developed as compared with the normal case, the presence/absence of the elevated eyebrows is judged on the basis of the image of the eyebrows in addition to the above-described judgment based on the image, thereby being capable of conducting a more accurate judgment as to the drooping eyelid. For example, if it is judged on the basis of the image of the eyebrows that the eyebrows are elevated, the judgment that the drooping eyelid is slight on the basis of the image of the eyes can be corrected to the judgment that the drooping eyelid is serious, or the judgment that the symptom is normal on the basis of the image of the eyes can be corrected to the judgment that the symptom is the slight drooping eyelid so far as it is judged that the symptom is normal but close to the drooping eyelid. Such a correction may be appropriately conducted as occasion demands.

If the oral consultation is also transmitted, the above-described judgment may be made taking the contents of the oral consultation data into consideration to produce the diagnosis data. For example, in the case where the contents that user's sleeping hours are short on a preceding day are included in the oral consultation data, the diagnosis data can be produced taking the above contents into consideration.

If the judgment in the judging section 223 is delicate, the judgment in the judging section 223 is reserved so as to hand the judgment to the doctor or another expert. In this case, the production of the following prescription data can be conducted by the doctor.

The diagnosis data is transmitted to the prescription data producing section 225. The prescription data producing section 225 produces the prescription data on the basis of the diagnosis data.

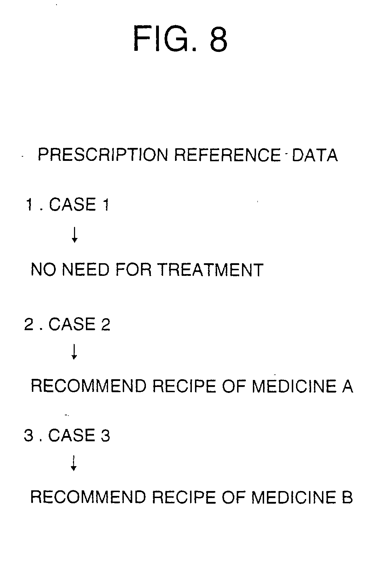

In the production of the prescription data, the prescription reference data related to the drooping eyelid recorded in the prescription reference data recording section 224 is used. The reference data is not particularly restricted if the reference data is used to conduct an appropriate and significant prescription for each of the drooping eyelid symptoms. An example of the reference data is shown in Fig. 8.

That is, if the diagnosis data is 1 indicative of "normal", data 1 indicating that no prescription is required is produced. If the diagnosis data is 1 indicative of "normal", data 1 indicating that no prescription is required is produced. If the diagnosis data is 2 indicative of "slight drooping eyelid", data 2 suggesting the dosage of a medicine A is produced. If the diagnosis data is 3 indicative of "serious drooping eyelid", data 3 suggesting the dosage of a stronger medicine B. Those data becomes the prescription data.

In the case where the oral consultation data is also transmitted, the above-described diagnosis data and prescription data are produced taking the contents of the interview prescription data into consideration.

For example, in the case where the contents that user's sleeping hours are short on a preceding day are included in the oral consultation data, even if the eyelid is slightly drooped, such a symptom can be judged as no disease. In particular, in the case where the sign of the drooping eyelid has not been found in the image data transmitted at the last two times, the drooping of the eyelid which is slightly developed can be judged as a short-period symptom that is caused by the shortage of sleep on a preceding day.

In the case where the oral consultation data is taken into consideration in the production of the prescription data, if the contents indicating that the sleeping hours are short are included in the oral consultation data, an instruction that sleeping hours should be increased as much as possible can be included in the prescription data.

The diagnosis data and the prescription data are transmitted to the terminal T used by the user or the like from the server S for use. In other words, the user can grasp his health state on the basis of the diagnosis data or the prescription data, or purchase and take a medicine. As occasion demands, the user can be subjected to a diagnosis by the doctor anew.

In this situation, the diagnosis data and the prescription data are transmitted from the transmit/receive section 210 of the server S through the network N. The terminal T receives the diagnosis data and the prescription data at its transmit/receive section 140.

The diagnosis data and the prescription data can be utilized by delivering those data from the server S to the drugstore Y. For example, it is possible that the drugstore Y extracts a person who appears to be anxious about his health on the basis of the diagnosis data, and send a direct mail to the person. Also, it is possible that the drugstore Y delivers the above-described medicine A or B indicated by the prescription data to the user, or asks the user about his intention to purchase those medicines by a direct mail, by using the prescription data. The utilization of the above diagnosis data and prescription data makes both of the user and the drugstore Y obtain advantages.

In this situation, the delivery of the data to the drugstore Y can be conducted by transmitting the data from the transmit/receive section 210 of the server S through the network N. In this case, the data can be received by a terminal not shown which is located at the drugstore Y. Also, the delivery of the data may be conducted through some recording medium, or may be conducted by means of a paper medium printed out from the server S.

The above-described process is regularly or irregularly repeated, and the diagnosis data and the prescription data are produced at times for use by the user. If the circumstances of the user changes, the diagnosis data and the prescription data also change with a time, with the result that the worthy diagnosis data and prescription data which meet the state of the user are always produced.

In the above-described embodiment, an example in which the prescription data is also utilized is described. However, if no prescription data is required, only the diagnosis data may be employed. In this case, the prescription reference data recording section 224 and the prescription data producing section 225 within the server S are not required.

### (Depression Diagnosis (1))

In the case where the user is subjected to depression, there are many cases in which the above-described drooping eyelid symptom is developed. Therefore, the diagnosis of the depression can be performed by conducting substantially the same treatment as that in the drooping eyelid.

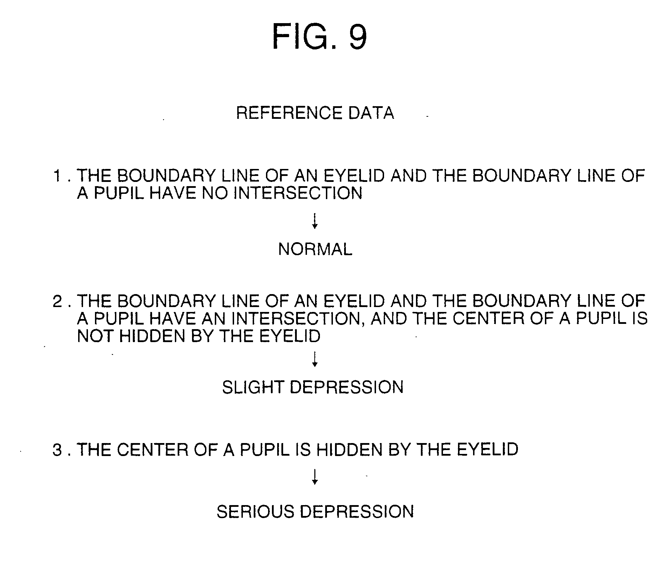

In other words, if the depression diagnosis data used when the judging section 223 produces the diagnosis data as shown in Fig. 9 is prepared, the depression diagnosis is conducted by execution of substantially the same treatment as that in case of the drooping eyelid diagnosis. In this case, the diagnosis data indicative of any one of the normal, the slight depression and the serious depression is produced. In the case where the oral consultation data is also transmitted, the diagnosis data is produced taking the oral consultation data into consideration as in the drooping eyelid diagnosis.

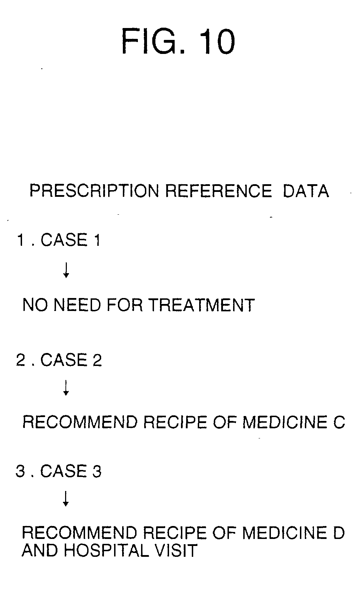

The prescription data producing section 225 produces the prescription data in accordance with the above-described diagnosis data. In this case, prescription reference data, for example, shown in Fig. 10 may be prepared. In this example, prescription data indicative of "No need for symptom", "Suggest the dosage of a medicine C" or "Suggest the. dosage of a medicine D and hospital visit" is produced.

How to utilize the diagnosis data and the prescription data by the user and the drugstore is substantially identical with that in the drooping eyelid diagnosis, and the above-described process can be regularly or irregularly repeated as in the drooping eyelid diagnosis.

### (Depression Diagnosis (2))

In the case where the user is subjected to depression, there has been known that a reaction of his pupils to a light stimuli is characteristic. On the basis of this phenomenon, the depression diagnosis can be executed by a method different from the above method.

Similarly, in this case, the user images his eyes by the imaging device C, but the image is a moving picture. In other words, a light constant in intensity is irradiated onto the eyes and thereafter a moving image indicative of a change in the diameter of the pupils in a given period of time is imaged. The image of the eyes is set within a constant range including user's eyes.

The image data thus produced is transmitted to the server S, as occasion demands, together with the oral consultation data.

The server S conducts the diagnosis of the depression on the basis of the image data (or both of the image data and the oral consultation data) transmitted from the terminal T through the network N.

The image data is transmitted to the image processing section 221, and then subjected to, for example, a binary or ternary coding in order to make it easy to detect the area of the pupil. In this example, the same image processing as that in the drooping eyelid diagnosis is conducted, to thereby produce the comparison image data.

Subsequently, the comparison image data is transmitted to the judging section 223. The judging section 223 compares the comparison image data with the reference data read from the reference data recording section 222, to thereby conduct a judgment on the basis of the comparison image data.

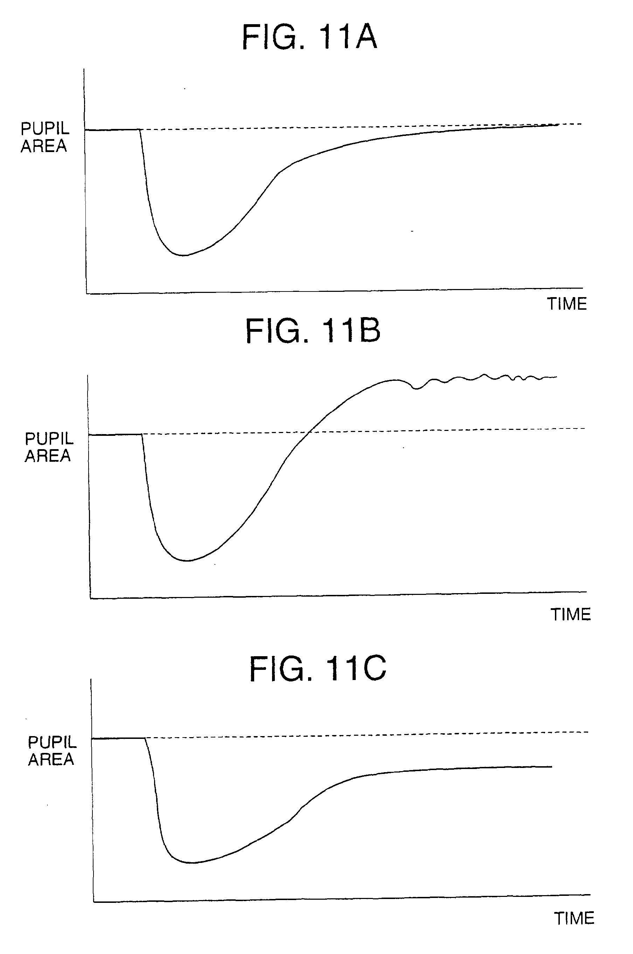

In this example, the reference data is related to the reaction patterns of the pupils, for example, as shown in Figs. 11A to 11C.

Figs. 11A to 11C show the reaction patterns of the pupils after a light has been irradiated onto the eyes. Fig. 11A shows a reaction pattern of the pupil of a person who is in a normal condition. In case where he is in the normal condition, the pupil that is made small in size by irradiation of a light is quickly returned to an original size. Fig. 11B shows a reaction pattern of the pupil of a person who is in an excessively strained condition. In case where he is the excessively strained condition, the pupil that is made small in size by irradiation of a light. becomes larger than the original size, and thereafter the pupil is repeatedly expanded and reduced without being not stabilized. Fig. 11C shows a reaction pattern of the pupil of a person who is subjected to depression. In the case where he is subjected to depression, the pupil that is made small in size by irradiation of a light has a tendency to be hardly expanded, and also may not be frequently returned to the original size. repeatedly expanded and reduced without being not stabilized.

The judging section 223 judges to which pattern in Figs. 11A to 11C the comparison image data is similar, and on the basis of this result, judges whether the user is subjected to depression or the like. In other words, if the comparison image data is similar to the pattern of Fig. 11A, diagnosis data indicating that the user is in the normal condition is produced, if the comparison image data is similar to the pattern of Fig. 11B, diagnosis data indicating that the user is in the excessively strained condition is produced, and if the comparison image data is similar to the pattern of Fig. 11C, diagnosis data indicating that the user is subjected to depression is produced.

The diagnosis data is transmitted to the prescription data producing section 225. The prescription data producing section 225 produces the prescription data on the basis of the diagnosis data.

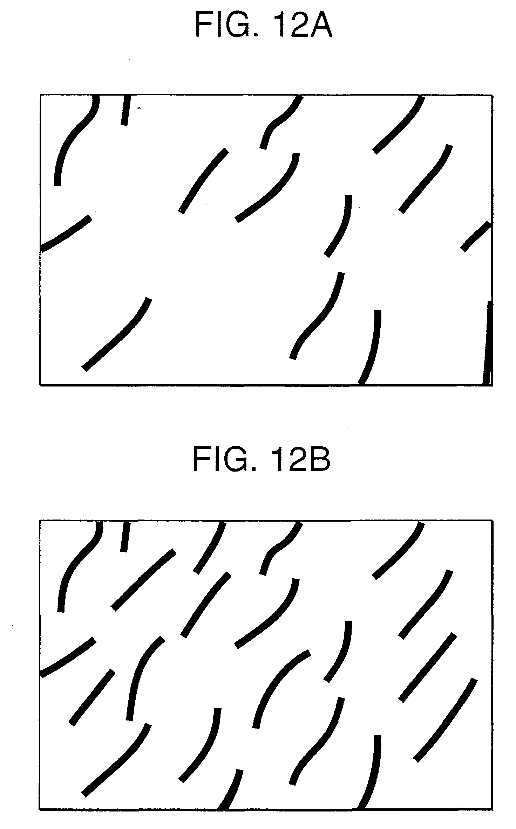

In the production of the prescription data, the prescription reference data recorded in the prescription reference data recording section 224 is employed. An example of the prescription reference data is shown in Figs. 12A and 12B.

The prescription data produced in this example will be described. If the diagnosis data is data indicating the user is in a normal condition, data 1 indicative of "No need for symptom" is produced. If the diagnosis data is data indicating that the user is in an excessively strained condition, data 2 indicative of "Suggest the dosage of a medicine E and hospital visit" is produced. If the diagnosis data is data indicating that the user is subjected to depression, data 3 indicative of "Suggest the dosage of a medicine F and hospital visit" is produced.

How to utilize the diagnosis data and the prescription data by the user and the drugstore is substantially identical with that in the drooping eyelid diagnosis, and the above-described process can be regularly or irregularly repeated as in the drooping eyelid diagnosis.

### (Insomnia Diagnosis and Autonomic Imbalance Diagnosis)

In the case where the user is subjected to insomnia or autonomic imbalance diagnosis, there are many cases where the above-described drooping eyelid symptom is developed. Also, those diseases almost supervene depression. Therefore, the insomnia diagnosis and the autonomic imbalance diagnosis can be conducted by executing the same process as that in the above-described depression diagnosis.

In case of the insomnia diagnosis, it is possible that diagnosis data is selectively produced from insomnia, slight insomnia, serious insomnia and so on, and prescription data related to an appropriate prescription for the selected diagnosis data is produced. As examples, prescription data including data related to a comment that suggests the dosage of an appropriate chemical agent or data related to a comment that suggests an exercise or a dietetic treatment may be produced. In order to achieve this process, the reference data and the prescription reference data may be so set as to produce the above-described diagnosis data and prescription data.

### (Hair Diagnosis)

People of both sexes has many worries about their hairs such as thin hair or the outgrowth of hair. Therefore, there is a great demand for the hair diagnosis which is easily conducted.

The diagnosis of the hair health condition is conducted by the following manner. In this example, a case in which the hair of user's head as the hair is diagnosed will be described.

In the case of diagnosing the hair of user's head, the user inputs information for inputting correspondence information from the input device T2 or the like. Also, the user images the hair of his head by the imaging device C to produce image data related to the image of the hair of his head. The image of the hair of his head is set within a constant range of a given portion of the head skin. In the case of imaging the given portion of the head skin, the positioning implement 10 shown in Fig. 2 can be employed. The positioning implement is employed in the above-described usage, to thereby simply image the same position.

Also, in this embodiment, the imaging of the hair of his head is conducted by a predetermined magnification.

The terminal T receives the image data produced by imaging the hair of the head through the imaging device C at the image data receive section 110. The data is transmitted to the server S through the network N together with additional information in response to a demand from the user.

As occasion demands, the user conducts operation for inputting oral consultation data including character information through the input device T2, to thereby produce the oral consultation data. In this situation, in inputting the oral consultation data, an image that prompts the selection of s can be displayed in the display device T1 as in the drooping eyelid diagnosis. In the case where the oral consultation has been inputted, the terminal T transmits the oral consultation data to the server S through the network N in addition to the image data.

The server S receives the image data (or both of the image data and the oral consultation data) transmitted from the terminal T by the transmit/receive section 210, and then diagnoses the hair of the head on the basis of that data.

As in the drooping eyelid diagnosis, the image data received by the transmit/receive section 210 is transmitted to the diagnosis section 220 to determine that the hair of the head is diagnosed by the correspondence judging section 226, and the determination is transmitted to the image processing section 221 and the judging section 223.

The image data of the hair of the head is transmitted to the image processing section 221. As occasion demands, the image processing section 221 conducts a given image process on the image data to produce the comparison image data.

For example, a given threshold value of brightness is set to subject the image to a binary or ternary coding, thereby being liable to conduct the subsequent judgment as to the image of the eyes. In this embodiment, the image of the hair of user's head is binary-coded so as to definitely distinguish the hair of the head from other portions. Fig. 12 shows an example of an enlarged image of the hair of the head displayed on the basis of the comparison image data.

The comparison image data which has been subjected to the above-described process as occasion demands is then transmitted to the judging section 223. The judging section 223 compares the comparison image data with the reference data read from the reference data recording section 222, to thereby conduct a judgment on the basis of the comparison image data.

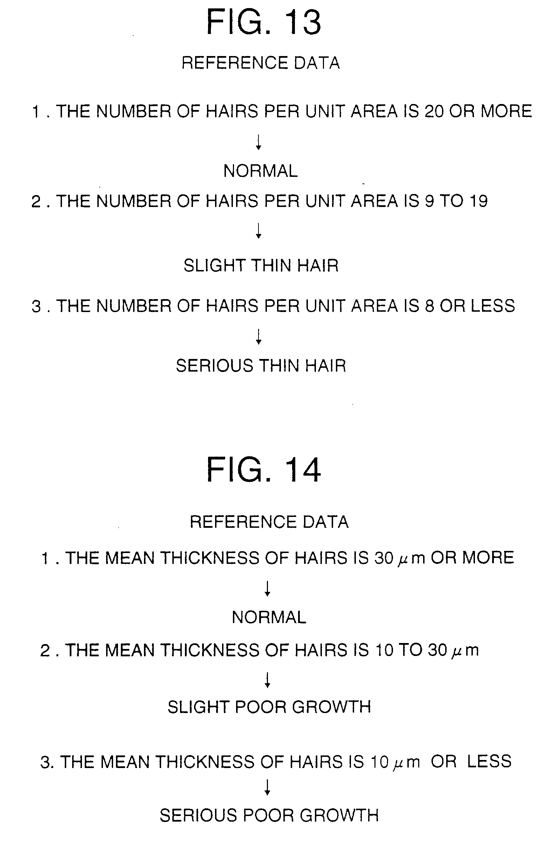

In this judgment, there is used the reference data related to the hair of the head recorded in the reference data recording section 222. The reference data is not particularly restricted if an appropriate judgment of the hair of the head is conducted. An example of the reference data is shown in Figs. 13 and 14.

The judging section 223 judges which reference shown in Figs. 13 and 14 the image shown in Fig. 12A or 12B is similar to, to thereby produce the diagnosis data.

In an example shown in Fig. 13, "normal", "slight thin hair", or "serious thin hair" is judged according to the number of the hair of the head with reference to the number of the hairs of the head per a unit area. In the example shown in Fig. 14, the growth state of the hair of the head is judged according to the thickness of the hair with reference to the mean thickness of the hair of the head. Specifically, "normal", "slight poor growth" or "serious poor growth" is judged.

The data related to the judgment result such as "normal", "slight thin hair", "serious thin hair" or "normal", "slight poor growth" or "serious poor growth" becomes diagnosis data.

In the case where the oral consultation data has been also transmitted, the above-described judgment is made taking the contents of the oral consultation data into consideration, to thereby produce the diagnosis data.

The diagnosis data is transmitted to the prescription data producing section 225. The prescription data producing section 225 produces the prescription data on the basis of the diagnosis data.

In the production of the diagnosis data, the prescription reference data related to the hair of the head recorded in the prescription reference data recording section 224 is used. The reference data is not particularly restricted if an appropriate and significant prescription is conducted for each of the hair states.

For example, in the case where the diagnosis data is indicative of "normal", data indicating that no treatment is required can be produced. In the case where the diagnosis data is indicative of "slight thin hair", data that suggests the coating of some medicine or presents shampoo or hair conditioner to be used can be produced. In the case where the diagnosis data is indicative of "serious thin hair", data that suggests the coating of a stronger medicine or presents shampoo or hair conditioner to be used can be produced. Those data produced on the basis of the diagnosis data becomes the prescription data.

In the case where the oral consultation data has been also transmitted, the above-described prescription data may be produced taking the contents of the oral consultation data into consideration.

The diagnosis data and the prescription data are transmitted from the server S to the terminal T used by the user for use. The user can grasp his hair health condition on the basis of the diagnosis data. Also, the user can purchase an appropriate medicine, and purchase the shampoo or hair conditioner on the basis of the prescription data.

Also, the prescription data can be delivered from the server S to the drugstore Y, the beauty goods shop B or the like for use. That is, the drugstore Y or the beauty goods shop B can conduct the same direct mail service as that in the drooping eyelid diagnosis by using the diagnosis data or the prescription data.

The above-described process is regularly or irregularly repeated to produce the prescription data at times, and the prescription data is used by the user.

### (Skin Aspect Diagnosis)

The diagnosis of the skin aspect health condition is conducted, for example, as follows. In this example, a case of diagnosing the degree of skin dry as the skin aspect will be described.

In the case of diagnosing the skin aspect, the user images the skin aspect by the imaging device C to produce the image data related to the image of the skin aspect. The image of the skin aspect is set within a constant range of a given portion of the skin. In the case of imaging the given portion of the skin, the positioning implement 10 shown in Fig. 2 can be employed. The positioning implement 10 is employed in the above-described usage, to thereby easily image the same position.

Also, in this embodiment, the skin aspect is imaged by a predetermined magnification.

The terminal T receives the image data produced by imaging the skin aspect through the imaging device C by the image data receive section 110. The data is transmitted through the network N in response to a demand from the user.

As occasion demands, the user conducts the operation for inputting the oral consultation data including the character information through the input device T2, to thereby produce the oral consultation data. In this situation, in inputting the oral consultation data, an image that prompts the selection of options can be displayed in the display device T1 as in the drooping eyelid diagnosis. In the case where the oral consultation has been inputted, the terminal T transmits the oral consultation data to the server S through the network N in addition to the image data.

The server S receives the image data (or both of the image data and the oral consultation data) transmitted from the terminal T by the transmit/receive section 210, and then diagnoses the hair of the head on the basis of that data.

As in the drooping eyelid diagnosis, the image data received by the transmit/receive section 210 is transmitted to the diagnosis section 220 to determine that the skin aspect is diagnosed by the correspondence judging section 226, and the determination is transmitted to the image processing section 221 and the judging section 223.

The image data of the skin is transmitted to the image processing section 221. As occasion demands, the image processing section 221 conducts a given image process on the image data to produce the comparison image data.

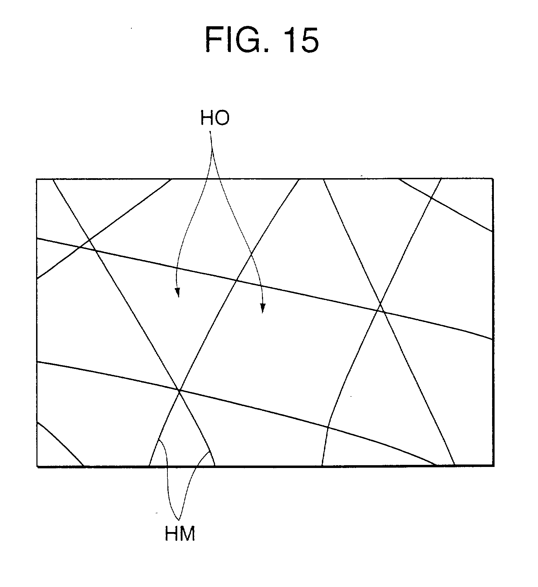

In this example, a given threshold value of brightness is set to subject the image to a binary coding, thereby being liable to conduct the subsequent judgment. Fig. 15 shows an example of an enlarged image of the skin indicated by the comparison image data. In Fig. 15, HM expressed as lines that extend lengthwise and breadthwise are grooves in the outer skin which are called "skin grooves", and the respective portions sectioned by the skin grooves HM are called "skin hill HO".

The comparison image data which has been subjected to the above-described process as occasion demands is then transmitted to the judging section 223. The judging section 223 compares the comparison image data with the reference data read from the reference data recording section 222, to thereby conduct a judgment on the basis of the comparison image data.

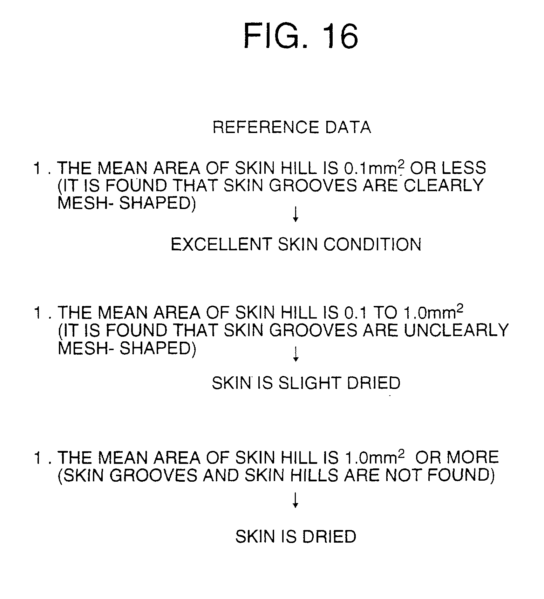

In this judgment, there is used the reference data related to the skin aspect recorded in the reference data recording section 222. The reference data is not particularly restricted if an appropriate judgment of the skin aspect is conducted. The reference data is shown, for example, in Fig. 16 on the basis of the size of the skin hill HO.

The health skin grooves HM extend lengthwise and beadthwise. However, the skin grooves HM of the bad health extends in only one direction, and the worse health skin has no skin groove HM per se. That is, the skin is more delicate with more moisturized health skin, and an area of the skin hill HO becomes small. The above-described reference data is used to judge the degree of the skin dry on the basis of the above knowledge.

The judging section 223 judges which reference shown in Fig. 16 the image shown in Fig. 15 on the basis of the comparison image data is similar to, to thereby produce the diagnosis data.

In an example shown in Fig. 16, "excellent skin condition", "skin is aging" or "skin is aged" is judged on the basis of the state of the skin groove HM or the size of the area of the skin hill HO.

The conclusion of "excellent skin condition", "skin is aging" or "skin is aged" obtained as a result of the judgment becomes diagnosis data.

In the case where the oral consultation data has been also transmitted, the above-described judgment is made taking the contents of the oral consultation data into consideration, to thereby produce the diagnosis data.

For example, the appropriate size of the area of the skin hill HO depends on age. Therefore, the judgment of "excellent skin condition", "skin is aging" or "skin is aged" is corrected on the basis of the data of ages included in the oral consultation data, thereby being capable of producing more accurate diagnosis data.

The diagnosis data is transmitted to the prescription data producing section 225. The prescription data producing section 225 produces the prescription data on the basis of the diagnosis data.

In the production of the diagnosis data, the prescription reference data related to the skin aspect recorded in the prescription reference data recording section 224 is used. The reference data is not particularly restricted if an appropriate and significant prescription is conducted for each of the skin aspects.

For example, in the case where the diagnosis data is indicative of "excellent skin condition", data indicating that a cosmetic A should be used can be produced. In the case where the diagnosis data is indicative of "skin is aging", data indicating that a cosmetic B should be used can be produced. In the case where the diagnosis data is indicative of "skin is aged", data indicating that a cosmetic C should be used and including a comment that proposes that a judgment should be made by an expert can be produced. Those data produced on the basis of the diagnosis data becomes the prescription data.

In the case where the oral consultation data has been also transmitted, the above-described prescription data may be produced taking the contents of the oral consultation data into consideration.

The diagnosis data and the prescription data are, for example, returned from the server S to the terminal T used by the user for use. That is, the user can determine a cosmetic to be purchased, or can ask for a further judgment by the expert on the basis of the prescription data.

Also, the diagnosis data and the prescription data can be delivered from the server S to the beauty goods shop B or the like for use. That is, the beauty goods shop B can conduct the same direct mail service as that in the drooping eyelid diagnosis or send an appropriate cosmetic by using the prescription data.

The above-described process is regularly or irregularly repeated to produce the prescription data at times, and the prescription data is used by the user.

As was described above, according to the present invention, the user can be subjected to simple medical care or medical treatment at any time not depending on the convenience of the doctor. Also, according to the present invention, since a fixed point is continuously observed, a precision in the medical care or medical treatment is kept to a high level although the doctor is not required in principle.

The foregoing description of the preferred embodiments of the invention has been presented for purposes of illustration and description. It is not intended to be exhaustive or to limit the invention to the precise form disclosed, and modifications and variations are possible in light of the above teachings or may be acquired from practice of the invention. The embodiments were chosen and described in order to explain the principles of the invention and its practical application to enable one skilled in the art to utilize the invention in various embodiments and with various modifications as are suited to the particular use contemplated. It is intended that the scope of the invention be defined by the claims appended hereto, and their equivalents.

## Claims

1. A diagnosis system, comprising:
a terminal device that receives image data obtained by imaging from a given imaging means that repeatedly images a position to be imaged which is located at a given position of a user's body; and
an information processing device which is connectable to said terminal device through a network;
wherein said terminal device includes a transmitting means for transmitting the image data produced by imaging the position to be imaged by said imaging means to said information processing device through said network;
wherein said image processing device includes a diagnosing means that conducts a given diagnosis on the basis of image data received from said terminal and produces diagnosis data pertaining to a diagnosis result; and
wherein said information processing device produces the diagnosis data in a time series manner on the basis of a plurality of image data produced by imaging the position to be imaged at given time intervals.

2. A terminal device which is connectable through a network to an image processing device having a diagnosing means that conducts a given diagnosis on the basis of image data received through the network to produce diagnosis data pertaining to a diagnosis result, said terminal device comprising:
a transmitting means that receives, from a given imaging means that repeatedly images a position to be imaged which is located at a given position of a user' s body, the image data produced by imaging the position to be imaged with the use of said imaging means by the user to transmit the image data to the information processing device through said network.

3. A terminal device which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result on the basis of the diagnosis result, said terminal device comprising:
a transmitting means that receives, from a given imaging means that repeatedly images a position to be imaged which is located at a given position of a user' s body, the image data produced by imaging the position to be imaged with the use of said imaging means by the user and the diagnosis data and transmits the image data and the diagnosis data to the information processing device through said network.

4. A method of transmitting diagnosis information which is executed by a terminal device that is connectable through a network to an information processing device having a diagnosing means that conducts a given diagnosis on the basis of image data received through the network to produce diagnosis data pertaining to a diagnosis result, said method comprising the steps of:
receiving image data produced, from a given imaging means that repeatedly images a position to be imaged which is located at a given position of a user's body, the image data produced by imaging the position to be imaged with the use of said imaging means by the user; and
transmitting the image data to the information processing device through the network.

5. A method of transmitting diagnosis information which is executed by a terminal device which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result, said method comprising the steps of:
receiving image data produced, from a given imaging means that repeatedly images a position to be imaged which is located at a given position of a user's body, the image data produced by imaging the position to be imaged with the use of said imaging means by the user and the diagnosis data; and
transmitting the image data and the diagnosis data to the information processing device through the network.

6. A recording medium that records, in a computer readable manner, a program for allowing a terminal device with a computer which is connectable through a network to an information processing device having a diagnosing means that conducts a given diagnosis on the basis of image data received through the network to produce diagnosis data pertaining to a diagnosis result to function as a transmitting device, said computer executing said program comprising the steps of:
receiving data produced, from a given imaging means that repeatedly images a position to be imaged which is located at a given position of a user's body, the image data produced by imaging the position to be imaged with the use of said imaging means by the user; and
transmitting the image data to the information processing device through the network.

7. A recording medium that records, in a computer readable manner, a program for allowing a terminal device with a computer which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result to function as a transmitting device, said computer executing said program comprising the steps of:
receiving image data produced, from a given imaging means that repeatedly images a position to be imaged which is located at a given position of a user's body, the image data produced by imaging the position to be imaged with the use of said imaging means by the user and the diagnosis data; and
transmitting the image data and the diagnosis data to the information processing device through the network.

8. A program recorded in a given recording medium in a computer readable manner, for allowing a terminal device with a computer which is connectable through a network to an information processing device having a diagnosing means that conducts a given diagnosis on the basis of image data received through the network to produce diagnosis data pertaining to a diagnosis result to function as a transmitting device, said computer executing said program comprising the steps of:
receiving data produced, from a given imaging means that repeatedly images a position to be imaged which is located at a given position of a user's body, the image data produced by imaging the position to be imaged with the use of said imaging means by the user; and
transmitting the image data to the information processing device through the network.

9. A program recorded in a given recording medium in a computer readable manner, for allowing a terminal device with a computer which is connectable through a network to an information processing device having a diagnosing means for conducting a given diagnosis on the basis of image data and oral consultation data pertaining to the diagnosis contents and including given character information which are received through the network to produce diagnosis data pertaining to a diagnosis result to function as a transmitting device, said computer executing said program comprising the steps of:
receiving image data produced, from a given imaging means that repeatedly images a position to be imaged which is located at a given position of a user's body, the image data produced by imaging the position to be imaged with the use of said imaging means by the user and the diagnosis data; and
transmitting the image data and the diagnosis data to the information processing device through the network.

10. An information processing device that is connectable through a network to a terminal device having a transmitting means that receives, from a given imaging means that repeatedly images a position to be imaged which is located at a given position of a user' s body, the image data produced by imaging the position to be imaged with the use of said imaging means by the user to transmit the image data through said network, said information processing device comprising:
wherein said image processing device includes a diagnosing means that conducts a given diagnosis on the basis of image data received from said terminal and produces diagnosis data pertaining to a diagnosis result; and
wherein said information processing device produces the diagnosis data in a time series manner on the basis of a plurality of image data produced by imaging the position to be imaged at given time intervals.

11. The information processing device as claimed in claim 10, wherein said diagnosing means comprises: an image processing means for conducting a given image process on the image data to produce comparison image data; reference data recording means for recording reference data for diagnosis; and a judging means for comparing the comparison image data with the reference data to conduct the given diagnosis.

12. An information processing device which is connectable through a network to a terminal device having a transmitting means that receives, from a given imaging means that repeatedly images a position to be imaged which is located at a given position of a user' s body, image data produced by imaging the position to be imaged by the user and oral consultation data pertaining to the diagnosis contents and including given character information and transmits the image data and the diagnosis data through said network, said information processing device comprising:
a diagnosing means that conducts a given diagnosis on the basis of the image data and the oral consultation data received from said terminal and produces diagnosis data pertaining to a diagnosis result;
wherein said information processing device produces the diagnosis data in a time series manner on the basis of a plurality of image data and oral consultation data produced by imaging the position to be imaged at given time intervals.

13. The information processing device as claimed in claim 12, wherein said diagnosing means comprises: an image processing means for conducting a given image process on the image data to produce comparison image data; reference data recording means for recording reference data for diagnosis; and a judging means for comparing the comparison image data and the oral consultation data with the reference data to conduct the given diagnosis.

14. The information processing device as claimed in claim 11 or 13, wherein the reference data includes data for conducting at least one of a depression diagnosis, an insomnia diagnosis, a drooping eyelid diagnosis, an autonomic imbalance diagnosis, a skin aspect diagnosis and a hair diagnosis, and said judging means conducts at least of the depression diagnosis, the insomnia diagnosis, the drooping eyelid diagnosis, the autonomic imbalance diagnosis, the skin aspect diagnosis and the hair diagnosis.

15. The information processing device as claimed in claim 11 or 13, wherein the reference data includes data necessary for conducting plural kinds of diagnoses; and
wherein correspondence information indicative of which diagnosis the image corresponds to is added to the image data, and said judging means conducts the plural kinds of diagnoses indicated by the correspondence information on the basis of the correspondence information received together with the image data.

16. The information processing device as claimed in claim 15,
wherein the reference data includes data for conducting at least one of a depression diagnosis, an insomnia diagnosis, a drooping eyelid diagnosis, an autonomic imbalance diagnosis, a skin aspect diagnosis and a hair diagnosis, and
wherein correspondence information indicative of which diagnosis the image corresponds to is added to the image data, and said judging means conducts any one of the depression diagnosis, the insomnia diagnosis, the drooping eyelid diagnosis, the autonomic imbalance diagnosis, the skin aspect diagnosis and the hair diagnosis indicated by the correspondence information on the basis of the correspondence information received together with the image data.

17. The information processing device as claimed in claim 10 or 12, further comprising a prescribing means for producing prescription data pertaining to a prescription suitable for the diagnosis result;
wherein said prescribing means produces the prescription data in accordance with the time series on the basis of the diagnosis data produced in accordance with the time series.

18. A method of producing diagnosis data which is executed by an information processing device which is connectable through a network to a terminal device having a transmitting means that receives, from a given imaging means that repeatedly images a position to be imaged which is located at a given position of a user' s body, the image data produced by imaging the position to be imaged by the user to transmit the image data through said network, said method comprising:
receiving the image data from said terminal; and
conducting a given diagnosis on the basis of the image data to produce the diagnosis data pertaining to the diagnosis result on the basis of the diagnosis result;
wherein said receiving step and said conducting step are repeatedly conducted on a plurality of image data produced by imaging the position to be imaged at given time intervals by the user to produce the diagnosis data in accordance with the time series.

19. A method of producing diagnosis data which is executed by an information processing device which is connectable through a network to a terminal device having a transmitting means that receives, from a given imaging means that repeatedly images a position to be imaged which is located at a given position of a user' s body, the image data produced by imaging the position to be imaged by the user and oral consultation data pertaining to the diagnosis contents and including given character information to transmit the image data and the oral consultation data through said network, said method comprising:
receiving the image data and the oral consultation data from said terminal; and
conducting a given diagnosis on the basis of the image data and the oral consultation data to produce the diagnosis data pertaining to the diagnosis result on the basis of the diagnosis result;
wherein said receiving step and said conducting step are repeatedly conducted on a plurality of image data produced by imaging the position to be imaged at given time intervals and the oral consultation data by the user to produce the diagnosis data in accordance with the time series.

20. A recording medium that records, in a computer readable manner, a program for allowing an information processing device with a computer which is connectable through a network to a terminal device having a transmitting means that receives, from a given imaging means that repeatedly images a position to be imaged which is located at a given position of a user' s body, the image data produced by imaging the position to be imaged with the use of said imaging means by the user to transmit the image data through said network to function as a diagnosis data producing device, said computer executing said program comprising the steps of:
receiving the image data from said terminal; and
conducting a given diagnosis on the basis of the image data and the oral consultation data to produce the diagnosis data pertaining to the diagnosis result;
wherein said receiving step and said conducting step are repeatedly conducted on a plurality of image data produced by imaging the position to be imaged at given time intervals to produce the plurality of diagnosis data in accordance with the time series.

21. A recording medium that records, in a computer readable manner, a program for allowing an information processing device with a computer which is connectable through a network to a terminal device having a transmitting means that receives, from a given imaging means that repeatedly images a position to be imaged which is located at a given position of a user' s body, the image data produced by imaging the position to be imaged with the use of said imaging means by the user and oral consultation data pertaining to the diagnosis contents and including given character information to transmit the image data through said network to function as a diagnosis data producing device, said computer executing said program comprising the steps of:
receiving the image data and the oral consultation data from said terminal; and
conducting a given diagnosis on the basis of the image data and the oral consultation data to produce the diagnosis data pertaining to the diagnosis result;
wherein said receiving step and said conducting step are repeatedly conducted on a plurality of image data produced by imaging the position to be imaged at given time intervals and the oral consultation data to produce the plurality of diagnosis data in accordance with the time series.

22. A program recorded in a recording medium in a computer readable manner, for allowing an information processing device with a computer which is connectable through a network to a terminal device having a transmitting means that receives, from a given imaging means that repeatedly images a position to be imaged which is located at a given position of a user' s body, the image data produced by imaging the position to be imaged with the use of said imaging means by the user to transmit the image data through said network to function as a diagnosis data producing device, said computer executing said program comprising the steps of:
receiving the image data from said terminal; and
conducting a given diagnosis on the basis of the image data and the oral consultation data to produce the diagnosis data pertaining to the diagnosis result;
wherein said receiving step and said conducting step are repeatedly conducted on a plurality of image data produced by imaging the position to be imaged at given time intervals to produce the plurality of diagnosis data in accordance with the time series.

23. A program recording in a recording medium in a computer readable manner, for allowing an information processing device with a computer which is connectable through a network to a terminal device having a transmitting means that receives, from a given imaging means that repeatedly images a position to be imaged which is located at a given position of a user' s body, the image data produced by imaging the position to be imaged with the use of said imaging means by the user and oral consultation data pertaining to the diagnosis contents and including given character information to transmit the image data through said network to function as a diagnosis data producing device, said computer executing said program comprising the steps of:
receiving the image data and the oral consultation data from said terminal; and
conducting a given diagnosis on the basis of the image data and the oral consultation data to produce the diagnosis data pertaining to the diagnosis result;
wherein said receiving step and said conducting step are repeatedly conducted on a plurality of image data produced by imaging the position to be imaged at given time intervals and the oral consultation data to produce the plurality of diagnosis data in accordance with the time series.
